# EUROPEAN PATENT APPLICATION

(11) **EP 1 010 980 A1**
(43) Date of publication of application: **21.06.2000**
(21) Application number: 99309908.4
(22) Date of filing: 09.12.1999
(51) Int. Cl.: G01N 33/53, G01N 33/68, G01N 33/577, G01N 33/563, G01N 33/546, G01N 33/574, G01N 33/74, G01N 33/58

(54) **Immunoassay of proteins**

(30) Priority: 17.12.1998 GB 9827845
(71) Applicant: Socolab S.A., 1300 Limal (BE)
(72) Inventor: Cambiaso, Cesar L., 1200 Brussels (BE); Masson, Pierre L., 1200 Brussels (BE)
(74) Representative: Wain, Christopher Paul

(57) **Abstract**

Immunoassay of a selected protein in a liquid sample containing the selected protein and one or more non-selected proteins is effected by employing denaturing means to denature the non-selected protein(s), under substantially nonreducing conditions, so as to lower or avoid interference from the non-selected protein. The denaturing is carried out under conditions such that thereafter the selected protein exhibits at least one antigenic determinant distinctive thereof and preferably capable of binding with a selective binding substance specific for the selected protein. Once denaturing has been terminated, and in the absence of the denaturing means, the antigenic determinant is assayed so as to determine the amount of the selected protein present in the liquid sample.

## Description

The present invention relates to the immunoassay of proteins. In an immunoassay of serum and / or other biological fluids for a protein of interest, there is commonly interference in the assay by other substances present in the serum or fluids. For example, complement factor and rheumatoid factor, both of which are endogenous to human blood serum, tend to interfere by reacting with antibodies used in certain assays. A further problem is that some patients also have antibodies directed against animal proteins including the immunoglobulins used as a source of specific antibodies for certain assays. Further, other proteins present in the serum can interfere through non-specific protein-protein interactions.

There have been various proposals made for avoiding interferences as described above. For example, interference by complement or rheumatoid factor can be overcome by using in the assay the F(ab')2 fragments of an antibody instead of the whole antibody. The F(ab')2 fragments are immunospecific to the protein under assay but do not react with complement or rheumatoid factor. This technique is described in GB 2 013 688, to which reference can be made for further details. A further example is where the effect of non-specific protein-protein interactions can be substantially reduced by the use of chaotropic agents as described in EP 0 038 181, again to which reference can be made for further details. Another way to avoid interferences in immunoassays can be the enzymatic digestion of proteins, as described in EP 0 051 985. A problem with such enzymatic digestion, however, is that not all protein antigens can be assayed after digestion. Such treatment can make some antigens completely non-reactive with their specific antibodies. There exists a need, therefore, to develop other procedures to substantially eliminate or lower interferences found in immunoassays.

The above problems are discussed in relation to interferences in immunoassays of protein antigens, however, interference from proteins is also a problem in assays of non-protein antigens. For example, not only can proteins interfere in the assay reaction, but also the antigen under assay may be bound to proteins and first has to be released therefrom before the assay can be effected. However, interferences of this sort may be overcome by first digesting the proteins using an enzyme such as pepsin (the non-protein antigen being unaffected). The non-protein antigens or haptens can also be released by denaturation of the proteins using alkaline or acid pH. The pH is then neutralised prior to the assay. This procedure is described for example by J. N Limet et al. in J. Med. Microbiol. 23 (1988) 37-45 or by V. Avesani et al. in J. Med. Microbiol. 40 (1994) 270-274). Their results suggest that their techniques may be of broad application in the assay of non-protein antigens (but not, of course, of protein antigens because denaturation is expected to prevent binding to their specific antibodies).

Denaturation of proteins is, of course, well known. A protein is said to be denatured when it has substantially lost its native structure. It is the three-dimensional arrangement (secondary, tertiary or quaternary structure) to which the term denaturation is generally applicable. A protein is usually denatured when exposed to non-physiological conditions of pH or temperature but also to certain salts or organic solvents at various concentrations. As a consequence of denaturation, most biological activities, e.g. antibody activity but also, for an antigen, the capacity to bind to antibody, generally disappear. After denaturation, precipitation frequently occurs during the neutralisation step, which may contribute to loss of biological activities for the proteins involved. Immunologists know that denaturation of a given protein can decrease its capacity to bind to antibodies raised against the native protein. In particular, the unfolding of the peptidic loops can cause a loss of antigenic determinants (that is, the portion of the antigen bound by a given antibody, also known as an epitope).

We have now surprisingly found, however, that the advantages obtainable by denaturation of interfering proteins in the assays of non-protein antigens can be obtained, together with other advantages, in the immunoassay of protein antigens. In particular, we have found that, very surprisingly, liquids containing two or more proteins, only one of which is to be assayed, can be subjected to substantial protein denaturation and the selected protein of interest thereafter assayed without interference from the other protein.

In one aspect, the invention provides a method of immunoassay of a selected protein in a liquid sample containing the selected protein and at least one non-selected protein which comprises: employing denaturing means to denature said non-selected protein under substantially non-reducing conditions, said denaturing means being substantially the sole means of lowering or avoiding interference from said non-selected protein; allowing said denaturing to occur under conditions such that thereafter, in the resulting mixture, said selected protein exhibits at least one antigenic determinant which is distinctive only thereof in said resulting mixture; terminating said denaturing and then in the absence of said denaturing means assaying said antigenic determinant and determining therefrom the amount of said selected protein present in said liquid sample.

In a method according to the present invention, the liquid sample is typically a biological sample, such as serum or the like.

Suitably, denaturing comprises exposing the non-selected protein to denaturing means as referred to above, such as at least one denaturing agent. The denaturing agent may comprise a chemical such as urea, guanidinium hydrochloride, organic solvents or the like, or the use of chaotropic agents. Other suitable denaturing means may include the application of sufficient heat to effect substantial denaturing. Preferably, however, denaturing involves a shift of pH and a typical denaturing agent comprises an acid or alkaline compound. The use of a pH shift to effect denaturing is advantageous, because it is easy to stop the denaturing activity using either an alkaline or acid compound depending of course on the nature of the denaturing agent employed. Ease of correction (or stopping) of denaturing conditions in this way is beneficial to thereafter allow binding of the binding substance to the selected protein. The extent of denaturation can depend not only upon the extent of the pH shift but also upon the structure of the non-selected proteins themselves and also the conditions of incubation, for example time, temperature, protein concentration, ionic composition of the medium, any subsequent neutralisation step or the like. Preliminary routine trials may be carried out to determine optimum conditions for a particular embodiment of a method according to the present invention. The invention is hereinafter described mainly with reference to pH shift but it is to be understood that denaturation by other means (though less preferred) can be used as discussed above.

The liquid sample is suitably mixed with the denaturing agent, such as an acid or alkaline solution, in the denaturing step. The denaturation is generally effected so as to substantially inactivate the non-selected protein or proteins, at least to an extent such that in the subsequent assay step, the non-selected protein or proteins do not interfere with the assay. Most preferably, the non-selected protein or proteins are substantially completely denatured. The extent of denaturing depends, inter alia, on the nature of the non-selected proteins, the importance of the pH shift, and the conditions under which the denaturation is effected.

In some instances, the selected protein is also partially or substantially completely denatured, provided that following denaturation the selected protein exhibits at least one antigenic determinant (in some cases at least two antigenic determinants) which is (or are) distinctive only thereof in the resulting mixture substantially as described above. In the case where at least two antigenic determinants are present, these may be the same or different. According to a particular embodiment of the present invention, the denaturation is effected under conditions such that the selected protein exhibits one or more antigenic determinants (hereinafter referred to as "identified antigenic determinants") that before the denaturing step were either not present, or not present in a form capable of binding with a selective binding substance specific for the selected protein and which can be used in the assay of the selected protein as hereinafter described in greater detail. The identified antigenic determinant or determinants may thus be formed as a result of the denaturing; alternatively, the identified antigenic determinant or determinants may have been already present on the selected protein prior to denaturing, but following denaturing may be present in a form capable of binding with a selective binding substance as referred to above. The identified antigenic determinant or determinants may then be used for the assay.

It is often beneficial to identify the antigenic determinant (or determinants) obtained following denaturing, as antisera and selective binding substances (such as antibodies) raised in response thereto can be used in a method according to the present invention. Typically, the determinant can be identified as follows. A sample of the selected protein is substantially denatured, and the resulting polypeptide chain is separated by chromatography. The substantially denatured protein is then injected into an antiserum-producing animal, for example, a rabbit. Normally a high titre and avidity antiserum will be obtained reacting strongly with the denatured protein but poorly or not at all with any native protein. The identified antigenic determinant is generally called "sequential" because it is directly dependent upon an amino acid sequence of the selected protein without much dependence on the three-dimensional arrangement thereof. Such a sequential antigenic determinant may be present in the original selected protein but may not have exhibited any antigenic activity at all, for example because it was so hidden in the molecule that it was substantially inactive due to steric hindrance or the like. The antisera formed in this way against the identified antigenic determinant may preferably be used in the immunoassay of the selected protein in the denatured serum. Similarly, selective binding substances, such as monoclonal antibodies, directed against the identified antigenic determinant in the denatured protein may also be used.

A method according to the present invention may, therefore, initially comprise identifying at least one antigenic determinant exhibited by the selected protein following the denaturing conditions employed in the present invention, which determinant is only distinctive for the selected protein in the resulting mixture following denaturing. A method according to the present invention may still further comprise raising antisera or a selective binding substance (typically one or more antibodies) specific for the identified antigenic determinant for subsequent use.

In a preferred aspect of the present invention, the selected protein comprises PSA (Prostatic Specific Antigen). In an alternative preferred aspect of the present invention, the selected protein comprises prolactin (PRL). In a further alternative preferred aspect of the present invention, the selected protein comprises a natural hormone. It is of course appreciated that many other proteins are suitable for use as the selected protein. It will also be appreciated that the suitability of the present invention for a given protein can be determined by routine experimentation, in which the conditions of denaturation can be adapted.

The non-selected protein or proteins may be any protein that might interfere with detection of the selected protein and that can be substantially denatured in a method according to the present invention. In the case where the liquid sample comprises serum, the non-selected proteins may for example be one or more of the following - complement factors, rheumatoid factor (IgM and IgG), albumin, prealbumin, various antibodies and the like.

Preferably, a method according to the present invention employs at least one selective binding substance specific for the selected protein, for use in determining the amount of the selected protein present in the original liquid sample. Generally, a method according to the present invention comprises, following terminating the denaturing and in the absence of the denaturing means, contacting the selected protein and the at least one selective binding substance specific for the selected protein, so as to assay the antigenic determinant or determinants exhibited by the selected protein and to determine therefrom the amount of the selected protein present in the original liquid sample.

It is also preferred that the selective binding substance comprises at least one antibody (preferably monoclonal) specific for the antigenic determinant exhibited by the selected protein following denaturation as carried out in a method according to the present invention. Preferably, in the case where two or more identified antigenic determinants substantially as described above are exhibited by a selected protein following denaturation the selective binding substance comprises at least two distinct monoclonal antibodies, and preferably the antibodies have been raised specifically against the said determinants again substantially as described above. Thus, for example, when the selected protein has been substantially denatured, we prefer to raise an antibody or antibodies against any identified antigenic determinant and to use the antibody or antibodies for the assay. Whole antibodies can be used, alternatively F(ab) or F(ab')2 fragments can be used as described in GB 2 013 688. The term F(ab) as used herein either refers to the F(ab) region of a whole intact antibody, or F(ab) fragments (e.g. F(ab')2 which have been separated from the F(c) part of an antibody). Hereinafter, references to the use of antibodies include references to the use of whole antibodies and of F(ab) or F(ab')2 fragments of antibodies.

There are many different assay techniques that can be used to measure the amount of selected protein present in the liquid sample. It is preferred to use a latex particle assay although other suitable assay techniques may be used. The preferred latex particle assay involves latex particle agglutination techniques. There are several such techniques, all of which are well known in the art.

The following are preferred latex particle agglutination techniques:
a denatured mixture containing the selected protein is mixed with latex particles carrying the selective binding substance (the latter typically comprising at least one antibody against one or more antigenic determinants of the former). Agglutination generally occurs to an extent dependent on the quantity of the antigenic determinant and the extent of agglutination can be measured either directly or, more preferably, by counting the unagglutinated latex particles;
a substantially denatured mixture containing a substantially denatured selected protein is typically mixed with a selective binding substance comprising one or more antibodies (against one or more determinants of the former) to form "antibody : antigen complexes". The complexes are preferably mixed with latex particles having a coating comprising IgG, in the presence of a limited amount of an agglutinator, such as rheumatoid factor or C1q factor of complement. The latex particles and the complexes compete for the limited amount of agglutinator, and the extent of agglutination is measured to provide a measure of the amount of the selected protein in the original sample;
a substantially denatured mixture containing a selected protein is mixed with latex particles bearing substantially the same antigenic determinant or determinants as the selected protein and a limited amount of antibody or antibodies thereto. The particles and the substantially denatured selected protein compete for the limited amount of antibody or antibodies. The agglutinating activity of the antibody or antibodies can be enhanced by addition of rheumatoid factor or monoclonal antibodies directed against specific determinants. The extent of agglutination can be measured to determine the amount of the selected protein in the original sample;
a substantially denatured mixture containing the selected protein is mixed with latex particles bearing substantially the same antigenic determinant or determinants as the selected protein and with smaller latex particles carrying antibody or antibodies thereto. The selected protein inhibits agglutination between the two different latex particles, and the extent of agglutination is measured to determine the amount of the selected protein in the original sample.

Examples of other suitable assay techniques include the following:
use of antibody or antibodies to one or more antigenic determinants (or other selective binding substance or substances), which are immobilised, for example, on a support wall, such as a tube or the like;

The assay for use in the invention can include any technique involving labels. Known techniques involving labels include radioimmunoassay, utilising a radiolabelled substance and antibodies; immunoradiometric assay, using radiolabelled antibodies; and any technique utilising isotopic, enzymatic, fluorimetric, luminescent, chemiluminescent or electroluminescent, cryptate or gold labels.

These named techniques, and others, are well known in the art.

For calibration purposes, the above techniques generally utilise standard results obtained by performing the assays on samples containing known amounts of the selected protein i.e. where the selected protein has been submitted to denaturating conditions substantially as described above. The general techniques are fully described, for example, in Cambiaso et al, 1977 J. Immunol. Methods 23,29, to which reference can be made for further detail. Generally the above assays employ, in place of whole antibody or antibodies, the F(ab) fragments thereof, e.g. the F(ab')2 fragments, as described in our UK specification No. 2013688.

The assay of proteins after a denaturation step, in addition to avoiding interferences, can offer other advantages. For example, some errors in prior art immunoassay of proteins can be due not to the technique itself but to bad conditions of storage or shipping of the samples. The protein to be assayed can be altered by changes in temperature or shift of pH due to bacterial contamination. A systematic denaturation of samples by acidification, in accordance with the present invention, can, however, decrease the risk of error. In underdeveloped countries when transport of samples can be critical, blood samples are sometimes collected on filter paper discs, which are then dried and sent to the laboratory, where such treatment can substantially denature some protein antigens and the present invention can avoid errors in the assay thereof. Further, determination of substantially denatured proteins rather than native protein molecules can also be useful when the assay has to be applied to biological fluids other than serum, e. g. intestinal juice or cellular extracts where proteases are abundant. After proteolysis, the remaining antigenic determinant or determinants are generally sequential and therefore are similar to those found in the denatured protein from which the peptides originate. Another application is the determination of antigens incorporated into cellular membranes, e.g. cytokine receptors. Their extraction requires detergents or reagents which tend to denature the molecules to be recovered for the assay.

In a further aspect of the present invention, there is provided a kit of parts comprising a liquid sample containing at least one selected protein and at least one non-selected protein; means for substantially denaturing said non-selected protein under substantially non-reducing conditions, whereby said denaturing means are employed as substantially sole means for lowering or avoiding interference from said non-selected protein; and means for assaying, in the absence of the denaturing means, at least one antigenic determinant exhibited by the selected protein in the mixture following denaturing, which antigenic determinant is only distinctive of the selected protein in the mixture, so as to determine the amount of said selected protein present in said liquid sample.

In a still further aspect of the present invention there is provided, in a method of immunoassay for detecting at least one selected protein or hapten in a liquid sample, use of denaturing means (preferably substantially as sole denaturing means) for lowering or avoiding interference resulting from non-selected protein present in said liquid sample, wherein the denaturing means are such that, once denaturing has been terminated and in the absence of the denaturing means, the amount of selected protein or hapten present in the liquid sample can be determined by assaying at least one antigenic determinant exhibited by the selected protein or hapten in the mixture following denaturing, which antigenic determinant is only distinctive of the selected protein or hapten in the mixture.

The present invention will now be further illustrated by the following Examples that do not limit the scope of the invention in any way.

### Example 1

PSA assay using sample denaturation by pH lowering.

### Principle:

Carboxylated latex particles are covalently coated with two monoclonal antibodies specific for human PSA. Agglutination is caused by PSA. Incubation of the sample at pH 1.3 destroys serum interferences. The denaturation conditions are such that the PSA remains immunologically reactive.

### Coating of latex particles

Monoclonal anti-PSA antibodies 8301 and 8311 were obtained from OY MEDIX BIOMEDICA, Finland. They were covalently coupled to latex particles of 0.8µ diameter, k-1080 from Rhône-Poulenc (Courbevoie, France) by the carbodiimide method as described by Galanti et al. J. Virol. Methods 1987; 18:215-224. The mixture of the two monoclonals (200 µl containing 50 µg of each in 20 mM phosphate buffer, pH 7) was added to 50 µl of a 10% (w/v) carbodiimide-activated latex suspension. After 12 h incubation at room temperature under shaking, the suspension was mixed for 10 min with 100 µl of 0.27 M glycine buffered saline (GBS) containing 0.1% polymerized bovine serum albumin (polyBSA from Boehringer, Manheim, Germany ) and then centrifuged for 10 min at 10 000 rev/min. The particles were resuspended in 1 ml of 1% cholate and sonicated for 10 sec. After 1 hr incubation at 37°C, the suspension was centrifuged again for 10 min at 10 000 rev/min. The pellet was then recovered in 1 ml of GBS containing 0.1% Tween, sonicated for 10 sec and again centrifuged. This step was followed by a double washing with 1 ml GBS containing 0.1% polyBSA, the particles being resuspended in 1 ml of 0.54 M GBS containing 2% polyBSA followed by a further 8.5 dilution with 0.54 M GBS containing 2% BSA.

When F(ab')2 fragments are used rather than the whole IgG, they may be coated on latex particles in a generally similar manner.

### Assay

Sample treatment: 25 µl of sample diluted 1/10 in GBS-BSA was mixed with 100 µl of 0.4 N HCl; incubation time = 10 min at 37°C.

25 µl of 2 M TRIS was added to neutralise the pH and then mixed with 25 µl of antibody-coated latex diluted 1/200 in GBS-BSA.

350 µl of the suspension, after a 15-fold dilution with GBS was then aspirated in an optical instrument adjusted to count the remaining unagglutinated particles, so giving an indirect measure of the amount agglutinated latex, which, in turn, was indicative of the quantity of PSA in the sample under assay.

### Results

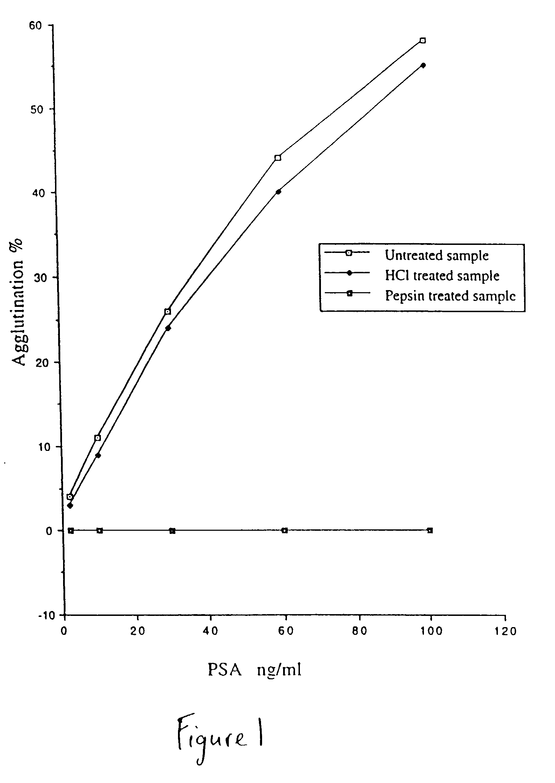

Fig. 1 shows a graph comparing the results of the agglutinating activity of PSA in an untreated sample, in the same sample after either HCl treatment or pepsin digestion. It can be seen that the HCl treatment slightly decreases the agglutinating activity of PSA but this effect is negligible when compared with that of the pepsin digestion, which abolishes the agglutination completely.

Table 1 illustrates the efficacy of the present invention to eliminate interferences caused by antibodies directed against mouse immunoglobulins. The PSA assay was applied to 6 human sera known to contain antibodies against mouse IgG and to 5 human sera devoid of such antibodies. As can be seen, when the samples were not treated by acid denaturation, the PSA values were found to be abnormally high. This also was true for the samples devoid of anti-mouse antibodies, indicating that interfering factors other than anti-mouse antibodies were present and could be inactivated by acid denaturation.

**Table 1.**

| Inactivation of interfering factors by acid treatment in the assay of PSA | | | | | |
|---|---|---|---|---|---|
| Sera containing anti-mouse antibodies No. | PSA values (ng/ml). Untreated samples | PSA values (ng/ml). Treated samples | Sera devoid of anti-mouse antibodies No. | PSA values (ng/ml). Untreated samples | PSA values (ng/ml). Treated samples |
| 1 | 23.2 | 1.5 | 1 | 13.3 | 2.4 |
| 2 | 61.2 | 1.9 | 2 | 10.9 | 1.3 |
| 3 | 129.8 | 1.3 | 3 | 13.4 | 1.8 |
| 4 | 17.6 | 1.1 | 4 | 11.7 | 1.6 |
| 5 | 81.3 | 0.7 | 5 | 11.8 | 1.1 |
| 6 | 42.6 | 3.7 | | | |

### Example 2

PRL assay using sample denaturation by pH lowering.

The technique was the same as for PSA. One mouse monoclonal antibody (N°090-10145) was from OEM Concepts (Torns River, New Jersey) and the second (N°5601) from OY MEDIX .

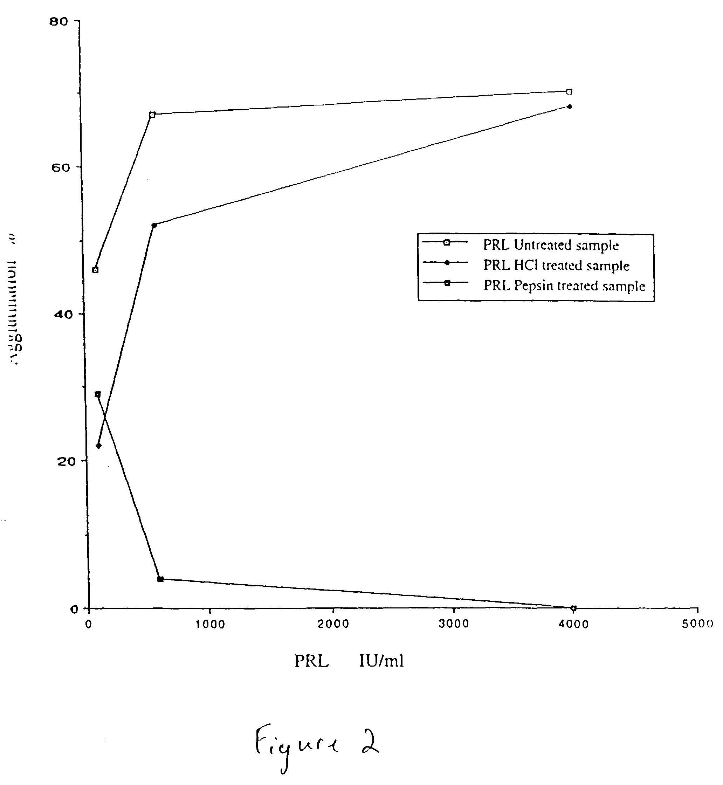

Fig. 2 shows a graph comparing the results of the agglutinating activity of PRL in an untreated sample, in the same sample after either HCl treatment or pepsin digestion. As in the PSA assay, the HCl treatment slightly decreased the agglutinating activity or PRL but this effect was negligible when compared with that of the pepsin digestion, which abolishes the agglutination completely.

Like Table 1, Table 2 shows the efficacy of the acid treatment on the interfering effects of anti-mouse antibodies.

**Table 2.**

| Inactivation of interfering factors by acid treatment in the assay of PRL | | |
|---|---|---|
| Sera containing antimouse antibodies No. | PRL values (ng/ml). Untreated samples | PRL values (ng/ml). Treated samples |
| 1 | 98.6 | 17.9 |
| 2 | 92.9 | 22.4 |
| 3 | 44.6 | 16.9 |
| 4 | >200 | 13.4 |
| 5 | 90.2 | 2.7 |
| 6 | >200 | 31.1 |

## Claims

1. A method of immunoassay of a selected protein in a liquid sample containing said selected protein and at least one non-selected protein; which comprises employing denaturing means to denature said non-selected protein under substantially non-reducing conditions, said denaturing means being substantially the sole means for lowering or avoiding interference from said non-selected protein; allowing said denaturing to occur under conditions such that thereafter, in the resulting mixture, said selected protein exhibits at least one antigenic determinant which is distinctive only thereof in said resulting mixture; terminating said denaturing and then in the absence of the denaturing means, assaying said antigenic determinant and determining therefrom the amount of said selected protein present in said liquid sample.

2. A method according to claim 1, wherein the liquid sample is serum, or any other biological fluid.

3. A method according to claim 1 or 2, wherein said denaturing means comprise an acid or alkaline compound.

4. A method according to any of claims 1 to 3, wherein the sample contains two or more non-selected proteins.

5. A method according to any of claims 1 to 4, wherein after denaturation, the antigenic determinant exhibited by the selected protein was hitherto either not present, or not present in a form capable of binding with a selective binding substance specific for the selected protein.

6. A method according to any of claims 1 to 5, wherein the selected protein comprises prostatic specific antigen.

7. A method according to any of claims 1 to 5, wherein the selected protein comprises prolactin.

8. A method according to any of claims 1 to 5, wherein the selected protein comprises a natural hormone.

9. A method according to any of claims 1 to 8, wherein the non-selected protein comprises any of complement factors, rheumatoid factor, albumin, prealbumin and antibodies.

10. A method according to any of claims 1 to 9, which comprises, following terminating the denaturing, in the absence of said denaturing means, contacting the selected protein and at least one selective binding substance specific for the selected protein, so as to assay the antigenic determinant exhibited by the selected protein and to determine therefrom the amount of the selected protein present in the liquid sample.

11. A method according to claim 10, wherein the selective binding substance comprises at least one antibody specific for the antigenic determinant exhibited by the selected protein following denaturation.

12. A method according to claim 11, wherein the selective binding substance comprises at least one monoclonal antibody.

13. A method according to claim 11 or 12, wherein the antigenic determinant is assayed by reacting with whole antibody.

14. A method according to claim 11 or 12, wherein the antigenic determinant is assayed by reacting with F(ab) or F(ab')2 fragments of antibody.

15. A method according to any of claims 1 to 14, wherein said antigenic determinant is assayed by a particle agglutination technique in which the amount of said selected protein is determined from the extent of agglutination of finely divided particles.

16. A method according to any of claims 1 to 14, wherein the assay uses enzymatic-fluorescent-, luminescent-, chemiluminescent- or radiolabelled molecules.

17. A kit of parts comprising a liquid sample containing a selected protein and at least one non-selected protein; means for substantially denaturing said non-selected protein under substantially non-reducing conditions, whereby said denaturing means are employed as substantially the sole means for lowering or avoiding interference from said non-selected protein; and means for assaying, in the absence of said denaturing means, at least one antigenic determinant exhibited by the selected protein in the mixture following denaturing, which antigenic determinant is only distinctive of the selected protein in the mixture, so as to determine the amount of said selected protein present in said liquid sample.

18. In a method of immunoassay for detecting at least one selected protein or hapten in a liquid sample, use of denaturing means for lowering or avoiding interference resulting from non-selected protein present in said liquid sample, wherein the denaturing means are such that, once denaturing has been terminated and in the absence of the denaturing means, the amount of selected protein or hapten present in the liquid sample can be determined by assaying at least one antigenic determinant exhibited by the selected protein or hapten in the mixture following denaturing, which antigenic determinant is only distinctive of the selected protein or hapten in the mixture.
